(19) Europäisches Patentamt
European Patent Office
Office européen des brevets

(11) Publication number: **0 456 948 B1**

# (12) EUROPEAN PATENT SPECIFICATION

(45) Date of publication of patent specification: **30.08.95**

(51) Int. Cl.[6]: **C07H 15/04**, A61K 31/70

(21) Application number: **90500129.3**

(22) Date of filing: **28.12.90**

## (54) Method for the preparation of polyphosphorylated derivatives of galactopyranosides with a potential insulin-like activity.

(30) Priority: **10.05.90 ES 9001306**

(43) Date of publication of application:
**21.11.91 Bulletin 91/47**

(45) Publication of the grant of the patent:
**30.08.95 Bulletin 95/35**

(84) Designated Contracting States:
**AT BE CH DE DK FR GB GR IT LI LU NL SE**

(56) References cited:
**EP-A- 0 131 993**
**EP-A- 0 354 323**
**US-A- 4 873 355**

(73) Proprietor: **EUROPHARMA, S.A.**
**Avda. de la Hispanidad, 21**
**E-28042 Madrid (ES)**

(72) Inventor: **Martin Lomas, Manuel**
**Avda. del Mediterraneo 24**
**E-28014 Madrid (ES)**
Inventor: **De La Paz, Jose Ma Mato**
**Tikal 1-2,**
**Chalet 125,**
**Burgo de Las Rozas**
**Las Rozas,**
**Madrid (ES)**

(74) Representative: **Elzaburu Marquez, Alberto et al**
**Sres. Elzaburu**
**Industrial Property Attorneys**
**Miguel Angel, 21**
**E-28010 Madrid (ES)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 456 948 B1

## Description

This invention is concerned with the preparation of di, tri, and tetraphosphorylated derivates of alkyl galactopyranosides of formula I

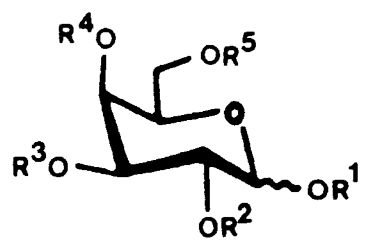

wherein
$R^1$ is C1-C8 alkyl and, at least two of $R^2$, $R^3$, $R^4$, and $R^5$ are

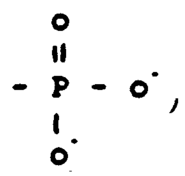

and the remaining rests $R^2$ to $R^5$ are hydroxyl groups.

Compounds of formula I are of interest as derivatives with a potential insulin-like activity.

Insulin is one of the most widely studied polypeptide hormones whose main cellular effects include the estimulation of glucogen, lipid and protein synthesis through the modulation of the metabolic pathways implicated in these processes, and the estimulation of cellular growth. The physiological effects of insulin include estimulation of transport of glucose, aminoacids and ions across the plasma membrane, regulation of the level of phosphorylation of proteins and enzymes controlling intermediary metabolism, such as glucogen phosporylase, hormone-sensitive lipase, and ATP-citrate-iase, and regulation of specific gene expression of several enzymes [R.M. Denton, in Advances in Cydic Nucleotide Research, 20 (1986). 293].

All these effects take place after insulin binds to its receptor, a cell surface glycoprotein constituted by two $\alpha$- and two $\beta$-subunits. Insulin binds to the $\alpha$-subunits which are linked tofether and to the $\beta$-subunits by disulfide bonds. After interaction with the insulin tyrosine residues of the $\alpha$-subunits are rapidly self-phosphorylated. It has been recently shown that this tyrosine kinase activity in the receptor is necessary for hormone action [O.M. Rosen, Science 237 (1987). 1452-1458].

Binding of insulin to its receptor estimulates the hydrolysis of a glycosyl phosphatidyl inositol (GPI) which generates a phosphooligosaccharide (POS) that mimicks and probably mediates an important number of biological actions of the hormone [A.R. Saltiel, J.A. Fox, P. Sherline and P. Cuatrecasas; Science 233 - (1986). 967-972; J.M. Mato, Cellular Signaling 1 (1989). 143-146] such as antilipolitic and lipogenic effects, nitogenic effect, and aminoacid transport POS also shows activity on a variety of enzymes such as piruvate dehidrogenase, cAMP-phosphodiesterase, cAMP-dependent protein kinase, and casein Kinase II [A.R. Saltiel, Endocrinology 120 (1987). 967-972; S. Alemany, J.M. Mato, P. Strälfors, Nature 330 (1987). 77-79; M. Villalba, K.L. Kelly, and J.M. Mato 968 (1988) 69-76].

The complete chemical structure of POS is presently unknown although the presence of inositol monophosphate linked to glucosamine, galactose, and phosphate groups has been reported [J.M. Mato, K.L. Kelly, A. Ables, and L. Jarret, J. Biol. Chem. 262 (1987) 2131-2137; J.M. Mato, K.L. Kelly, A. Abler, L. Jarret, B.E. Corkey, J.A. Carhed, D. Zopf, Biochem. Biophys. Res. Commun. 152 (1987) 1455-1462; M. Villalba, K.L. Kelly, J.M. Mato, Biochem. Biophys. Acta 968 (1988) 69-76], It has been shown that the biological activity of POS depends on the presence of phosphate groups [J.M. Mato, K.L. Kelly, A. Abler and L. Jarrett, J. Biol. Chem. 262 (1987) 2131-2137],
In this invention the synthesis of phosphorylated derivatives of D-galactose and the investigation of the possible "insulin-like" activity of these compounds analyzing their effect on aminoacid transport, glucogen synthesis and protein kinase activity, is reported.

From US-A-4.873.355 granted on Oct. 10, 1989, assigned to E.I. Du Pont de Nemours and Company there is known a process of synthesis of isomers of myo-inositol poli(phosphates), also adapted for the regioselective synthesis of any cyclitol phosphate, wich uses a phosphitylation reaction as the key step. Although this phosphitylation reaction and the corresponding phosphitylating agents had been previously employed in other systems, they had never been applied to the problem of producing inositol phosphates and other cyclitol phosphates.

From J. Org. Chem. 46 (1981) 3936-3938 there is known an improved procedura for ruthenium tetraoxide catalyzed oxidations of organic compounds. This improved system derives from the addition of acetonitrile to the traditional $CCl_4/H_2O$ solvent system for ruthenium tetraoxide catalyzed oxidations. There are described some applications of this procedure to olefins, alcohols, aromatic rings and ethers.

The synthesis of phosphorylated derivatives or galactose has been carried out from specifically protected derivatives of alkyl α-and β-D-galactopyranosides.

Phosphorylation of polyhydroxylated compounds is problematic mainly because of steric crowding and easy formation of cyclic phosphates from ricinal diols. However recent interest in inositol phosphate chemistry has brought about a number of new phosphorylation methods. These methods include either activation of alcohol function with strong bases and subsequent application of tetrabenzyl pyrophosphate as a phosphorylating agent [Y. Watanabe, H. Nakahira, M. Bunya, S. Ozaki, Tetrahedrom Lett. 28 (1987) 4179-4180; S.J. Debolms, J.P. Vacca, J.R. Huff, Ibid 28 (1987) 4503-4504; D.C. Billington, R. Baker, J. Chem. Soc. Chem. Commun. (1987) 1011-1013; J.P. Vacca, S.J. Debolms, J.R. Huff, J. Am. Chem. Soc. 109 - (1987) 3478-3479] or phosphytilation mainly with A,A-dialkyl dibenzyl phosphoramidite and subsequent oxidation [J.L. Meek, F. Davidson, F.W. Hobbs Jr. J. Am. Chem. Soc. 110 (1988) 2317; C.E. Dreef, R.J. Tuinman, C.J.J. Tlie, G.A. Van der Marel, J.H. Van Boom, Red. Trav. Chim. Pays-Bas 107 (1988) 395; K.L. Yu, B. Fraser-Reid Tetrahedron Lett. 29 (1988) 976; C.E. Dreef, G.A. Van der Marel, J.H. Van Boom, Red. Trav. Chim. Pays-Bas 106 (1987) 161; C.B. Reese, J.G. Ward, Tetrahedron Lett. 28 (1987) 2309; A.M. Cooke, B.V.L. Potter, R. Gigg, Tetrahedrom Lett. 28 (1987) 2305; Y.C. Lin, C.S. Chen, Tetrahedron Lett. 30 - (1989) 1617; G. Baudin, B.I. Glänzer, K.S. Swaminathan, A.Vasella, Helv. Chim. Acta 71 (1988) 1367].

We now have used this latter approach to prepare a series of di-, tri-, and tetraphosphorylated derivatives of alkyl α- and β-D-galactopyranosides. The phosphites resulting from the reaction of conveniently protected α- and β-D-galactopyranosides with dibenzyl-N,N-dialkyl phosphoramidite have been oxidized conventionally with m-chloroperbenzoic acid [see references above]. However we have observed that the reaction is most conveniently carried out in the presence of sodium periodate and catalytic amounts of ruthenium trichloride in a biphasic system [P.H.J. Carlsen, T. Katsuki, V.S. Martin, K.B. Sharpless, J. Org. Chem. 46 (1981) 3936].

The insulin-like activity of the alkyl α-and β-D-galactopyranoside di-, tri-, or tetraphosphate sodium salts has been investigated with respect ot transport of aminoacids, effect on protein-kinase A, effect on casein-kinase II, and synthesis of glucogen. The results indicate that compounds of this type have a potential interest for the treatment of diabetes particularly in the cases of insulin intolerance.

The following examples illustrate the synthetic processes and show the biological activity measurements with some of the products prepared.

## Examples

### Metyl α-D-galactopyranoside 2,6-diphosphate sodium salt (1).

Methyl 4,6-benzylidene-α-D-galactopyranoside (0.115 g, 0.39 m moles) in dry acetonitrile (3 mL) and dry dichloromethane (3 mL) was treated with anhydrous tetrazol (0.25 g, 3.6 m mol) and dibenzyl N,N-diisopropyl phosphoramidite (0.64g, 1.83 m mol)

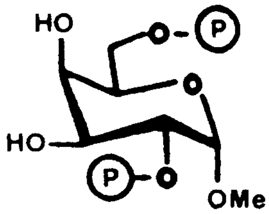

1

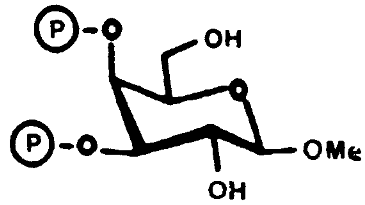

2

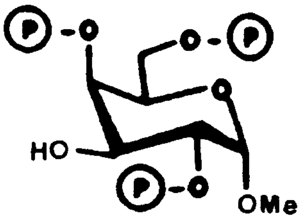

3

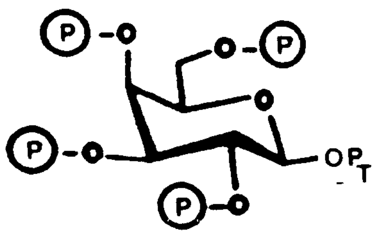

4

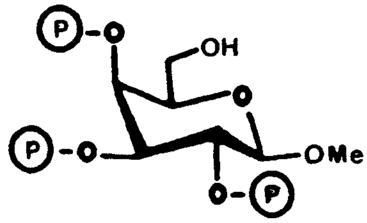

5

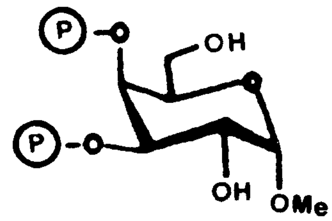

6

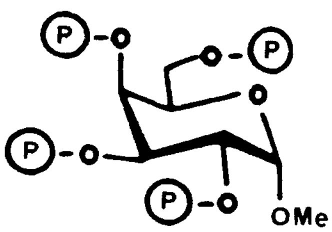

7

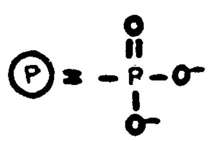

with stirring for 1 hr. After cooling to O°C metachloroperbenzoic acid (0.6 g, 3.5 m mol) in dry dichloromethane (3 mL) was added dropwise. The mixtere was then kept for 1.5 hr. at room temperature with stirring, concentrated in vacuo and the residue was washed with 10% aqueous sodium thiosulfate, and 10% aqueous sodium carbonate, dried and purified through a silica gel column eluting with ethyl acetate-hexane (1:2 ----> 3:1 v/v to give 0.270 g of the corresponding phosphate, $[\alpha]_D + 84.9°$ (c 1.0 chloroform).

The above phosphate (0.270 g) in methanol was treated with hydrogen in the presence of 10% palladium on charcoal for 4 hr. The mixture was filtered, and treated with Amberlite® C6-120 ($Na^+$) resin to give compound 1 (0.060 g) $^1$H-R,MN (300 $MH_2$, $D_2O$) $\delta$: 3.43 (5, 3H, OMe), 3.76 (d, 2H, $J_{5,6} = J_{5,6'}$ 6.1 $H_2$, H-6+H'-6'), 3.94 (m, 1H, H-5), 4.18 (m, 1H, $J_{3,4}$ 2, $J_{4,5}$<1 $H_2$, H-4), 4.34 (m, 1H, H2 + H-3), 5.01 (d, 1H, $J_{1,2}$ 2.9, H-1). $^{13}$C R.M N. ($D_2O$, 70 MHz): 56.24 (OMe), 62.38 (C-6), 70.06 and 71.62 (C-4, C-5), 72.30 and 74.25 (C-2 and C-3), 99.61 (C-1).

## Methyl β-D-galactopyranoside 3,4-diphosphate sodium salt (2)

Methyl 3,4-O-isopropylidene β-D-galactopyranoside (1.8 g) in dry N,A-dimethylformamide was treated with sodium hudride with stirring for 1 hr. Benzyl bromide (3.7 mL) was added dropwise with at °C. Stirring

was continued for 18 hr. at room temperature. After cooling to O°C, methanol was first added and then water and the mixture was extracted with chloroform. The chloroform extract was evaporated in vacuo to give a residue of crude methyl 2,6-di-O-benzyl 3,4-O-isopropyledene-β-D-galactopyranoside (2g, 63%) which was used in the next step withouth further purification.

The above compound (2g) in 30% aqueous acetic acid (30 mL) was heated at 100° for 2 hr. After removal of the solvent the residue was passed through a bed of silice gel to afford 1.74g (94%) of methyl 2,6-di-O-benzyl-β-D-galactopyranoside.

The above compound (0.10 g, 0.26 m mol) in a mixture of dry dichloromethane (2 mL) and dry acetonitrile (2 mL). Dry tetrazol (0.160 g, 2.4 m mol) and dibenzyl-N,N-diisopropyl phosphoramidite (0.414 g, 1.62 m mol) were added and the mixture stirred for 1 hr at room temperature. Water , (3.5 mL), sodium metaperiodate (0.330 g, 1.56 m mol) and ruthenium trichloride trihydrate (2.7 mg, 0.010 m mol) were added and stirring continued for 1 hr. The mixture was extracted with dichloromethane (15 mL) and the extract was washed with water, dried and evaporated in vacuo to give a residue which was purified on a column of silica gel. Elution with ethyl acetate-hexane (2:1 v/v) afforded 0.2g of the corresponding phosphate $[\alpha]_D$ + 18.5° (c 0.7 chloroform).

The above compound (0.270 g) in methanol (30 mL) was treated with hydrogen in the presence of 10% palladium on charcoal for two hours. The mixture was filtered on Celite®, and evaporated in vacuo. The residue was passed through a column of Amberlite CG-120 ($Na^+$) to give 2.

## Methyl α-D-galactopiranoside 2,4,6-tri-phosphate sodium salt: (3).

Methyl α-D-galactopyranoside (0.6 g, 3.1 m mol) in acetonitrile (65 mL) was refluxed overnight with dibutyltin oxide (1 g, 4.02 mmol) and 3Å molecular sieves (4 g) at benzyl bromide (18.7 g, 0.11 mmol) and tetrabutylammonium bromide (0.49 g, 1.53 mmol) were added and refluxing continued for 2 hr. After cooling the mixture was filtered and the filtrate concentrated in vacuo to give a residue which was purified on a silica gel column. Elution with ethyl acetate gave pure methyl 3-O-benzyl-α-D-galactopyranoside (0.430 g).

The above compound (0.20 g, 0.70 mmol) in a 1:1 mixture of dry dichloromethane-acetonitrile (14 mL) was treated as previously described with dry tetrazol (0.65 g. 9.3 mmol) and dibenzyl N,N-diisopropyl phosphoramidite (1.7 g, 4.86 mmol) for two hours at room temperature. Water (10 mL), sodium metaperiodate (1,35 g, 6.34 mmol) and ruthenium chloride trihydrate(0.011 g, 0.042 mmol) were added and the mixture keept at room temperature for 2 hr work-up as above described for 2 gave the corresponding pure triphosphate (0.47 g) $[\alpha]_D$+49.2° (c 1.0 chloroform).

The above compound was hidrogenated in the presence of 10% palladium on charcoal for two hours. The mixture was then filtered on Celite® and the filtrate evaporated in vacuo to give a residue which was treated with Amberlite® CG-120 ($Na^+$) in the usual manner to give 3 (80 mg). $^1$H-R.M.N (300 MHz, $D_2O$): δ 5,o3 (d, 1H, $J_{1,2}$ 3.1 Hz, H-1), 4.59 (m, 1H, $J_{2,3}$ 9, $J_{2,P}$ 10 Hz), 4.00 (m, 4N, H-3, H-5, H-6, H-6'), 3.43 (s, 4H, OMe). $^{13}$C-R.M.N. (70 MHz, $D_2O$): 99.5 (C-1) 75.4 (C-4), 73.8 (C-2) 70.8 (dd, C-3 or C-5) 69.7 (dd, C-5 or C-3), 65.6 (d, C-6), 56.5 (OMe).

## Propyl β-D-galactopyranoside 2,3,4,6-tetraphosphate sodium salt (4).

Alkyl β-D-galactopyranoside (0.05 g, 0.23 mmol) in a 1:1 mixture of dichloromethane-acetonitrile (4 mL) was treated with tetrazol (0.28 g, 4 mmol) and dibenzyl-N,N-diisopropyl phosphoramidite (0.72 g). After two hours m-chloroperbenzoic acid (0.704 g, 4.08 mmol) in dichloromethane (4 mL) was added dropwise at O°C. The mixture was kept for two hours at room temperature. After conventional work-up , 0.150 g of the corresponding tetraphosphate were obtained, $[\alpha]_D$+13° (c 0.97 chloroform).

The above compound was hydrogenated and treated with Amberlite® CG-120 ($Na^+$) as usual to give 4. $_1$H-R.M.N (300 MHz, $D_2O$) δ : 4.65 (dd, 1H, $J_{3,4}$ 3.0, $J_{4,P}$ 10.2 Hz, H-4), 4.60 (d, 1H, $J_{1,2}$ 7.6, H-1), 4.27 (m, 1H, $J_{3.4}$ 3.0, $J_{3,P}$ 9 Hz, H-3), 4.17 (m, 1H, $J_{2.3}$ 9, $J_{2,P}$ 9 Hz, H-2), 3.88 (m, 3H, H-5, H-6, and H-6'), 3.83 (m, 1H), 3.66 (m, 1H), 1.61 (m, 2H) 0.91 (t, 3H, Me), $^{13}$C-R.M.N. (70 MHz, $D_2O$): 103.1 (d, $J_{1,P}$ 5.2, C-1), 75.8 (t, C-2), 74.0, 74.1, 77.0 (C-3, C-4 and C-5), 73.8 (O-$CH_2$), 65.1 (d, C-6, 23.6 ($CH_2$), 11.0 (Me).

## Methyl β-D-galactopyranoside 2,3,4-triphosphate sodium salt (5).

Methyl 3,4,O-isopropylidene-β-D-galactopyranoside (0.45 g, 1.5 mmol) in toluene (25 mL) was refluxed with tributyltin oxide (1.36 g, 1.1 equiv.) and 3Å molecular sieves (2.5 g) for 18 hr. Benzyl bromide (2.5 mL) and N-methyl-imidazole were then added and the mixture refluxed for 24 hr. After the usual work-up, treatment of the resulting product with 40% aqueous acid at 90°C for 20 min. afforded pure. The above compound (0.098 g) in a 1:1 mixture of dichloromethane-acetonitrile (7 mL) was phosphitylated with dibenzyl N,N-diisopropyl phosphoramidite (0.850 g, 2.43 mmol) in the presence of tetrazole (3.25 mg, 4.65 mmol) as previously described. The oxidation step was carried out using sodium metaperiodate (0.670 g, 6.3 mmol), ruthenium chloride trihydrate (5 mg, 0.042 mmol) and water (3.5 mL). The hydrogenolysis step was performed as usual to give a residue which after treatment with Amberlite® CG-120 ($Na^+$) resin afforded pure 5 (79 mg). $^1$H-R.M.N. (300 MHz, $D_2O$): δ 4.59 (dd, 1H, $J_{3,4}$ 3, $J_{4,P}$ 10.3 Hz, H-4), 4.47 (d, 1H, $J_{1,2}$ 7.7 Hz, H-1), 4.25 (m, 1H, $J_{3,P}$ 9.5 Hz, H-3), 4.13 (m, 1H, $J_{2,3}$ 9.5, $J_{2,P}$ 9Hz, H-2), 3.76 (m, 3H, H-5, H-6 and H-6'), 3.54 (s, 3H, OMe). $^{13}$C-R.M.N.= 104.2 (C-1, d, $J_{C1-P}$ 3,9 Hz), 76.7 (C-3), 75.6 (C-4), 75.3 (C-2), 73.5 (C-5), 61.6 (C-6), 58.6 (OMe).

## Methyl-α-D-galactopyranoside 3,4-diphosphate sodium salt (6).

Methyl 2,6-di-O-benzyl-α-D-galactopyranoside (0.662 g, 1.77 mmol), prepared from methyl 3,4-O-isopropylidene-α-D-galactopyranoside by conventional benzylation and acid hydrolysis as described above for compound 2, was treated with dibenzyl N,N-diisopropyl-phosphoramidite (2.74 g, 7.9 mmol) and tetrazole (1.01 g, 1.44 mmol) in a 1:1 mixture of dichloromethane-acetonitrile (10 mL) as in the above cases. Oxidation was performed using sodium metaperiodate (1.82 g, 8.5 mmol) and ruthenium chloride trihydrate (7 mg, 0.026 mmol) and hydrogenolysis with hydrogen in the presence of 10% palladium on charcoal in methanol (20 mL) and buffer acetic-sodium acetate 1M. pH (4.5). $^1$H-N.MR data (300 MHz, $D_2O$): δ 4.90 (d, 1H, $J_{1,2}$ 3.4 Hz, H-1), 4.70 (dd, 1H, H-4), 4.36 (m, 1H, H-3), 4.02 (m, 1H, H-2), 3.98 (m, 1H, H-5), 3.77 (m, 2H, H-6, H-6'), 3.44 (s, 3H, OMe). $^{13}$C-N.M.R. data ($D_2O$, 70 MHz): 100.7 (C-1), 74.7 (C-3), 74.0 (C-4), 71.5 (d, J 3Hz, C-5 or C-2), 68.6 (d, J 6.6 Hz, C-2 or C-5), 62.2 (C-6), 56.7 (OMe).

## Methyl α-D-galactopyranoside 2,3,4,6-tetraphosphate sodium salt (7).

Methyl α-D-galactopyranoside (0.212 g, 1,1 mmol) was phospitylated as usual with dibenzyl N,N-diisopropyl phosphoramidite (3.61 g, 10.4 mmol). The oxidation step was carried out with m-chloroperbenzoic acid (2.9 g, 16.7 mmol) and the hydrogenolysis conventionally. $^1$H-R.M.N. (300 MHz, $D_2O$) data: δ 5.05 (d, $J_{1,2}$ 3.3 Hz, H-1), 4.75 (m, 1H, H-4), 4.46 (m, 1H, H-3), 4.40 (m, 1H, H-2), 4.17 (m, 1H, H-5), 4.04 (m, 2H, H-6 and H-6'), 3.44 (s, 3H, OMe). $^{13}$C-N.M.R. (70 MHz, $D_2O$): 99.9 (C-1) 75.0 (d, J 5.8 Hz, C-4), 73.5 (m, C-3), 72.5 (t, J 5.7, C-2), 70.7 (dd, J 4.0, J 8.2 Hz, C-5), 65.9 (d, J 4.6, C-6), 56.8 (OMe).

## Biological activity of compounds 1 - 7.

The biological activity of the phosphorylated derivatives of galactose is shown in Table 1. The effects on aminoacid transport, protein kinase A (cAMP-dependent) activity, and casein kinase II activity were determined according to J.M. Mato et al., Biochem.Biophys.Res.Commun. 152 (1987) 1455-1462; M. Villalba et al, Biochem.Biophys.Acta 968 (1988) 69-76; and S. Alemany et al., J.Biol.Chem. (1990) in the press.

Both compounds 2 and 4 estimulate the synthese of glucogen at concentrations 2-10 M.

TABLA I. Biological activity of phosphorylated alkyl α-and β-D-galactopyranosides.

| Compound | Aminoacid Transport | | LDH | | Protein Kinase A | | Protein Kinase II | |
|---|---|---|---|---|---|---|---|---|
| Control | | 100 | | 11 | | 100 | | 100 |
| 1 | (400 μM) | 63 | (400 μM) | 13 | (100 μM) | 100 | (400 μM) | 87 |
| 2 | ( " ) | 78 | ( " ) | 14 | ( " ) | 100 | ( " ) | 95 |
| 3 | ( " ) | 91 | ( " ) | 18 | ( " ) | 97 | ( " ) | 79 |
| 4 | ( " ) | 220 | ( " ) | 17 | ( " ) | 80 | ( " ) | 39 |
| | | | | | (200 μM) | 60 | | |
| | | | | | (400 μM) | 24 | | |
| 5 | ( " ) | 81 | nd | | ( " ) | 67 | ( " ) | 103 |
| 6 | ( " ) | 72 | nd | | ( " ) | 77 | ( " ) | 113 |
| 7 | ( " ) | 93 | nd | | ( " ) | 44 | ( " ) | 118 |
| POS | ( 20 μM) | 225 | ( 20 μM) | 15 | ( 20 μM) | 20 | ( 20 μM) | 40 |
| INSULIN | (100 μM) | 225 | (100 μM) | 13 | -- | | -- | |

## Claims

**1.** Process for obtaining polyphosphorylated galactose derivatives having potential insulin activity of formula I

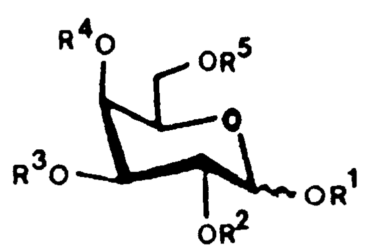

wherein
$R^1$ is C1-C8 alkyl and, at least two of $R^2$, $R^3$, $R^4$, and $R^5$ are

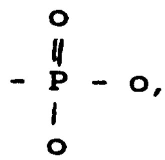

and the remaining rests $R^2$ to $R^5$ are hidroxyl groups, characterized in that, in a first step, alkyl $\alpha$- or $\beta$-D-galactopyranosides or 3,4-isopropylidene- or 4,6-benzylidene $\alpha$ or $\beta$-D-galactopyranosides containing two or three free hydroxyl groups are subjected to phosphitylation using phosphitylating agents; in a second step, the partially phosphitylated derivatives thus obtained are subjected to oxidation with a ruthenium trichloride catalyst and sodium periodate in a bi-phasic system; and in the third step the phosphorylated derivatives are deblocked to give the corresponding free phosphates which are isolated in sodium salt form.

**2.** Process according to claim 1, characterized in that the deprotection of the resulting phosphorylated derivates is carried out through acidic hydrolysis or catalytic hydrogenation.

**Patentansprüche**

**1.** Verfahren zur Herstellung von phosphorylierten Galaktosederivaten mit einer möglichen Insulin-ähnlichen Wirksamkeit, der Formel (I)

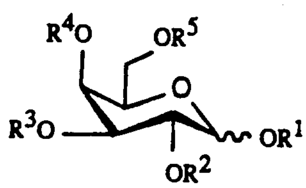

worin $R^1C_1$-$C_8$ Alkyl bedeutet und mindestens zwei von $R^2$, $R^3$, $R^4$ und $R^5$ die Bedeutung der Formel

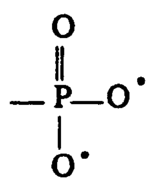

haben, und die restlichen Reste $R^2$ bis $R^5$ Hydroxylgruppen sind, dadurch gekennzeichnet, dass in einem ersten Schritt Alkyl $\alpha$- oder $\beta$-D-Galaktopyranoside oder zwei oder drei freie Hydroxylgruppen enthaltende 3,4-Isopropyliden- bzw. 4,6-Benzyliden- $\alpha$- oder $\beta$-D-Galaktopyranoside unter Verwendung von Phosphitierungsmittel phosphitiert werden; in einem zweiten Schritt, die so erhaltene, teilweise phosphitierte Derivate mit einem Rutheniumtrichloridkatalysator und Natriumperiodat in einem Zweiphasensystem oxydiert werden; und in einem dritten Schritt die phosphitierten Derivate zu den entsprechenden freien Phosphaten, die in einer Natriumsalzform isoliert werden, entblockiert werden.

**2.** Verfahren gemäss Anspruch 1, dadurch gekennzeichnet, dass die Entschützung der erhaltenen phosphorylierten Derivate mittels saurer Hydrolyse oder mittels katalytischer Hydrierung durchgeführt wird.

**Revendications**

**1.** Procédé pour obtenir dérivés phosphorylés de galactose ayant une activité potentielle analogue à celle de l'insuline de formule (I):

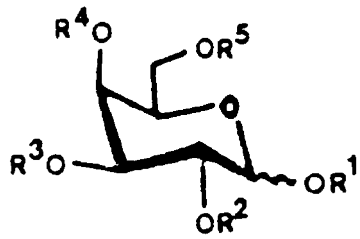

dans laquelle:

$R^1$ représente un groupe alkyle en $C_1$ à $C_8$, et au moins deux des symboles $R^2$, $R^3$, $R^4$ et $R^5$ sont

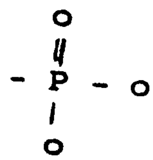

et les autres motifs $R^2$ à $R^5$ sont des groupes hydroxile, caracterisé en ce que, dans la première étape, un alkyl $\alpha$- ou $\beta$-D-galactopyrannoside ou 3,4-isopropylidene - ou 4,6-benzylidene $\alpha$- ou $\beta$-D galactopyrannoside contenant deux ou trois groupes hydroxile libres sont soumis à une phosphitylation en utilisant des agents phosphytilatants; dans la second étape, les dérivés partiellement phosphitylatés ainsi obtenus sont soumis à une oxydation avec catalyst de trichlorure de ruthénium et periodate de sodium dans un système à deux phases; et dans la troisième étape les derivés phosphorylés sont débarassés (déprotégés) pour donner les phosphates libres correspondants qui sont isolés en forme du sel de sodium.

**2.** Procédé selon la revendication 1, caracterisé en ce qu'on effectue par hydrolyse acide ou hydrogénation catalytique la déprotection des dérivés phosphorylés résultants.